# EUROPEAN PATENT APPLICATION

(11) **EP 1 486 188 A1**
(43) Date of publication of application: **15.12.2004**
(21) Application number: 03708652.7
(22) Date of filing: 17.03.2003
(51) Int. Cl.: A61F 13/15

(54) **DISPOSABLE WEARING ARTICLE**

(30) Priority: 20.03.2002 JP 2002077516
(71) Applicant: Zuiko Corporation, Settsu-shi, Osaka 566-0045 (JP)
(72) Inventor: KURATA, Shuhei, c/o ZUIKO CORPORATION, Settu-Shi, Osaka 566-0045 (JP)
(74) Representative: Schwabe - Sandmair - Marx
(86) International application number: PCT/JP2003/003188
(87) International publication number: WO 2003/077812

(57) **Abstract**

A disposable worn article of the present invention includes: a core portion (2) including a first end portion (21), a central portion (20) and a second end portion (22); and a waist portion (1) crossing the core portion (2), wherein: the waist portion (1) includes an elastic member for contracting/expanding the waist portion (1) in a longitudinal direction thereof, and first and second sheets interposing the elastic member therebetween; a portion of the waist portion (1) and a portion of the first end portion (21) of the core portion (2) are bonded together in a bonding section (4); and a contraction force from the elastic member is reduced in the bonding section (4).

## Description

### TECHNICAL FIELD

The present invention relates to disposable worn articles such as diapers and pants.

### BACKGROUND ART

A worn article disclosed in Japanese Laid-Open Patent Publication No. 6-30963 includes waist gathers along a waist portion so that the worn article fits around the waist of the wearer. A core portion covering the crotch of the wearer includes therein an absorbent so that bodily discharge can be absorbed.

An end portion of the core portion is bonded to the waist portion, whereby the end portion of the core portion contracts in the longitudinal direction of the waist portion due to the contraction force from the waist gathers of the waist portion.

If the core portion contracts, the core portion becomes corrugated and gives a stiff feel to reduce the wearability, or the core portion does not closely fit to the crotch of the wearer, causing a leak of bodily discharge.

The core portion hinders the waist gathers from contracting freely, thereby decreasing the facility in putting the worn article on the wearer and also deteriorating the fit of the waist portion to the waist.

Thus, it is an object of the present invention to provide a disposable worn article capable of overcoming these drawbacks.

### DISCLOSURE OF THE INVENTION

In order to achieve the object above, a disposable worn article according to a first aspect of the present invention includes: a core portion including a first end portion, a central portion and a second end portion; and a waist portion crossing the core portion. The waist portion includes an elastic member for contracting/expanding the waist portion in a longitudinal direction of the waist portion, and first and second sheets interposing the elastic member therebetween. A portion of the waist portion and a portion of the first end portion of the core portion are bonded together in a bonding section. A contraction force from the elastic member is reduced or inactivated in the bonding section.

A disposable worn article according to a second aspect of the present invention includes: a core portion suitable for covering a crotch of a wearer; and a waist portion capable of fitting to a waist of the wearer. The core portion includes a central portion with an absorbent, and first and second end portions located on opposite longitudinal ends of the core portion with respect to the central portion. The waist portion is attached to the first end portion so as to cross the core portion. The waist portion includes an elastic member for contracting/expanding the waist portion in a width direction of the core portion. A portion of the waist portion and a portion of the first end portion of the core portion are bonded together in a bonding section. A contraction force in the bonding section is smaller than a contraction force from waist gathers formed by the elastic member.

In a worn article of the present invention, the elastic member of the waist portion contracts the waist portion in the longitudinal direction thereof, thereby forming waist gathers in the waist portion. The elastic member for forming the waist gathers is not substantially provided in the bonding section where the waist portion and the core portion are bonded together, whereby the bonding section will not be contracted in the width direction of the core portion. Therefore, the first end portion of the core portion will not be creased or give a stiff feel due to the waist gathers.

In the present invention, the contraction force from the bonding section is smaller than that from the elastic member for forming the waist gathers, whereby the force with which the bonding section contracts the core portion in the width direction is smaller than that in a conventional worn article.

Thus, in the worn article of the present invention, although the waist portion is contracted/expanded in the longitudinal direction thereof due to the contraction force from the elastic member for forming the waist gathers, the bonding section itself does not include waist gathers, whereby the end portion of the core portion will not give a stiff feel, and the bulky core portion will not hinder the contraction of the waist gathers. Thus, the present invention provides an improved wearability and an improved fit.

In the present invention, "the contraction force from the elastic member being reduced or inactivated" may mean that no elastic member is present in the bonding section, and may also mean that the contraction force from the elastic member present in the bonding section is smaller than that from the waist gathers formed by the elastic member present in areas other than the bonding section.

Preferred examples of methods for reducing the contraction force of the elastic member in the bonding section are as follows.
(1) An elastic member provided on a sheet is cut off, together with the sheet, at at least one position during the production of the worn article of the present invention. In such a case, even if the elastic member is interposed between two sheets, the elastic member, which has been cut off during the production, contracts on the surfaces of the sheets. Thus, after the production, further contraction of the elastic member will not occur in the bonding section. Note that in order to prevent the contraction of the cut-off elastic member interposed between the two sheets, it is preferred that the adhesive for fixing the elastic member is not applied near the bonding section. Alternatively, the amount of the adhesive may be reduced near the bonding section.
(2) At least a portion of an elastic member provided on a sheet is melted together with a portion of the sheet and then solidified during the production of the worn article of the present invention. If the elastic member breaks off, the contraction force of the elastic member can be reduced for the same reason as that with the method (1) above. Moreover, even if the elastic member does not break off, the composition or the physical properties of the elastic member will be altered by heat, thereby reducing the contraction force of the elastic member. Moreover, the contraction force of the elastic member is reduced even by melting of a portion of the elastic member. Note that the methods (1) and (2) above can be combined together.

In the present invention, it is only required that the elastic member forming the waist gathers do not contract the waist portion in the bonding section. For example, as long as the elastic member is provided in the bonding section in a non-stretched (contracted) state, the elastic member may extend entirely across the length of the bonding section, or the elastic member may extend generally across the entire length of the waist portion while the elastic member is not divided.

If the elastic member is divided at a position in the longitudinal direction of the waist portion so as not to extend entirely across the bonding section, it is possible to produce a core portion in which the elastic member for forming the waist gathers does not substantially exist in the area of the bonding section itself. In such a case, the bonding section and the waist gathers are arranged so as not to overlap with each other.

In the present invention, an elastic member that does not form so-called "waist gathers" may be provided generally across the entire length of the waist portion. An example of an elastic member that does not form waist gathers may be, for example, an elastic member that is arranged along an edge of the cover material, which forms the inner surface of the waist portion.

In the present invention, a portion of the waist portion and the entirety of the first end portion of the core portion may be bonded together in the bonding section. Note however that in an article in which the waist portion is bonded only to a portion of the first end portion, as compared with an article in which the waist portion is bonded to the entirety of the first end portion, the area where no elastic member is present can be made smaller, whereby the waist gathers can be provided over a greater extent of the waist portion.

Moreover, a member forming the waist portion and the core portion may further be bonded together in an area other than the bonding section, i.e., in an area other than the first end portion. For example, a member forming the waist portion and a member forming the core portion may further be bonded together near the central portion of the core portion.

Moreover, in the present invention, the core portion includes an absorbent for absorbing bodily discharge. The absorbent may be, for example, fluff pulp obtained by fiberizing (crushing) pulp with a fiberizer (crusher) into a fibrous form, or a material obtained by mixing the fluff pulp with superabsorbent polymer particles (absorbent polymer) and letting it deposit into a cotton-like form, and the absorbent has a liquid absorbing property.

Moreover, the elastic member may be an rubber thread or a resin tape having elasticity or a net-shaped member such as Rebound. Where a rubber thread is used as the elastic member, a plurality of rubber threads may be arranged parallel to one another.

### BRIEF DESCRIPTION OF THE DRAWINGS

The present invention will be understood more clearly from the following description of preferred embodiments taken in conjunction with the accompanying drawings. Note however that the embodiments and the drawings are merely illustrative and should not be taken to define the scope of the present invention. The scope of the present invention shall be defined only by the appended claims. In the accompanying drawings, like reference numerals denote like components throughout the plurality of figures.

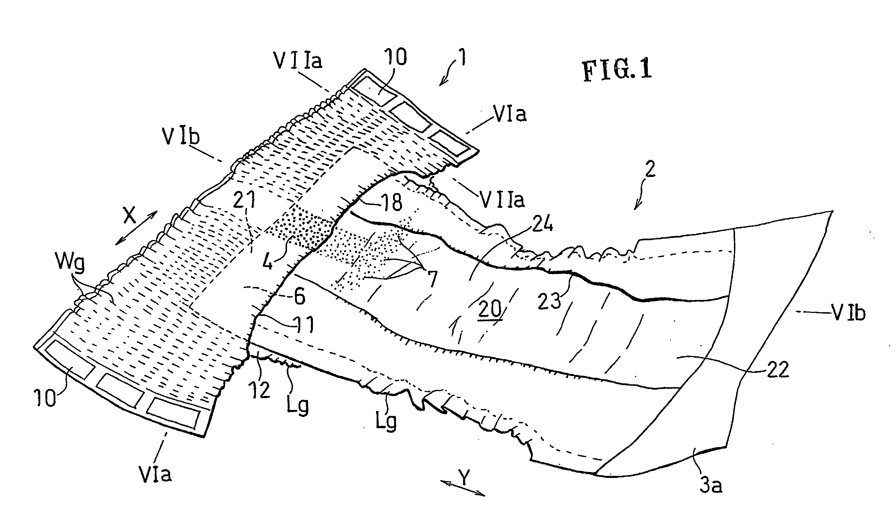

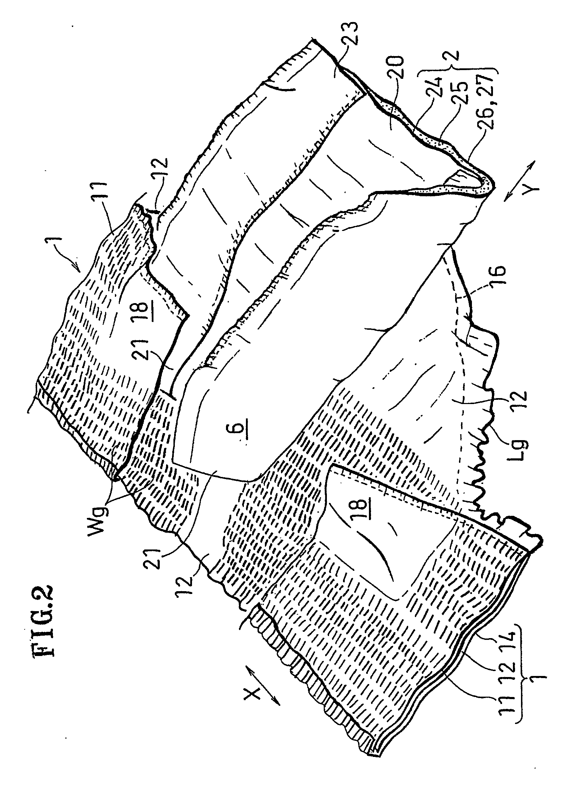

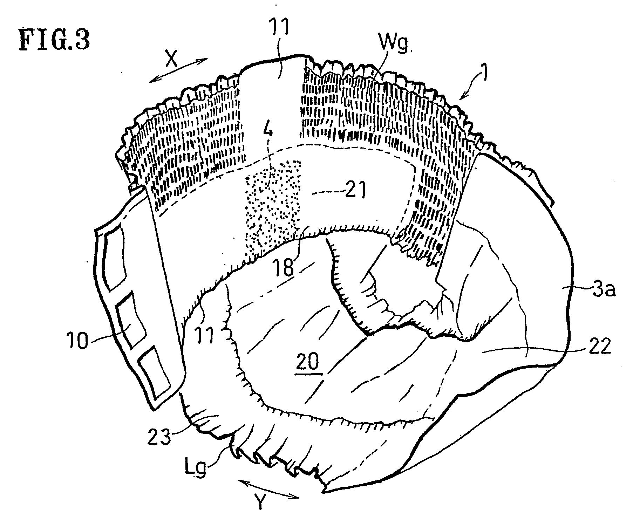

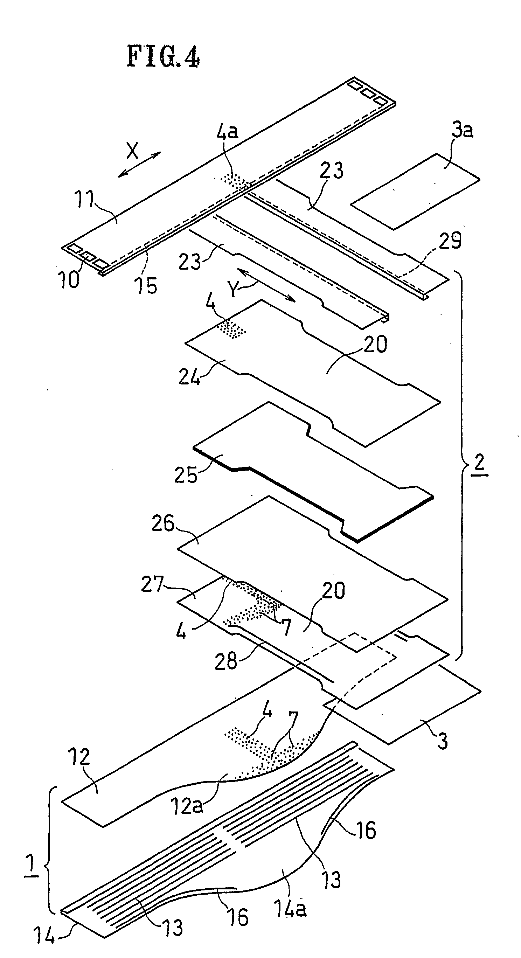

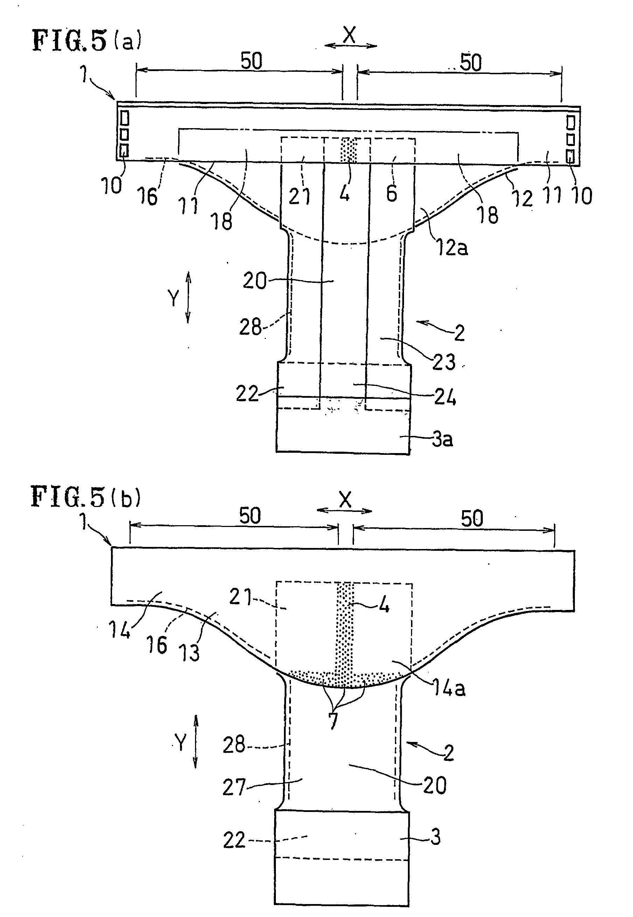

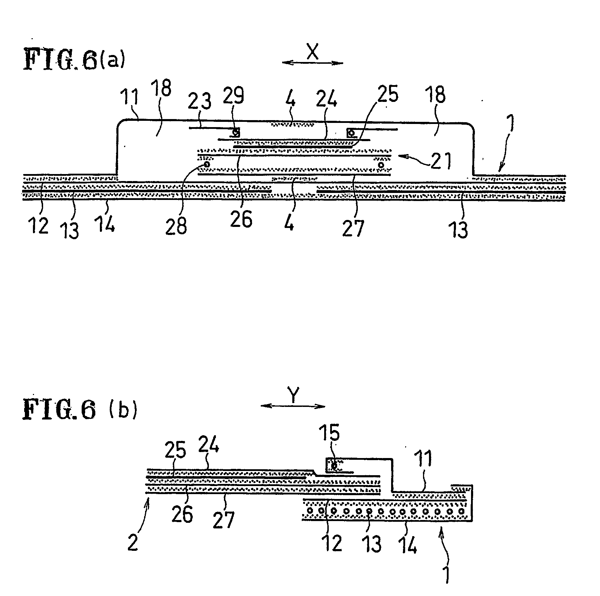

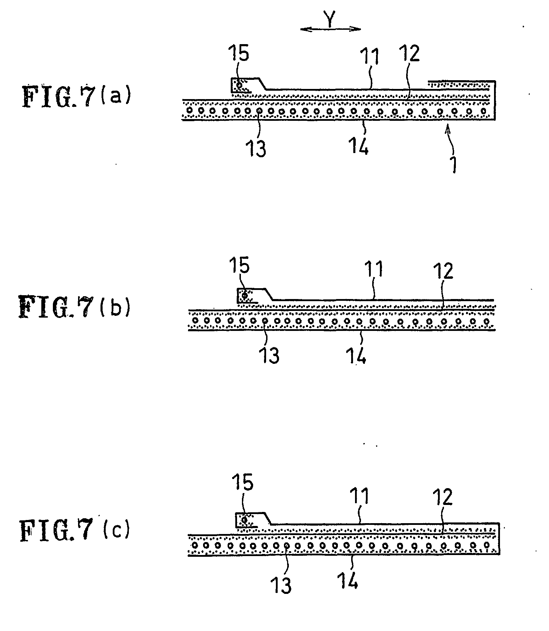

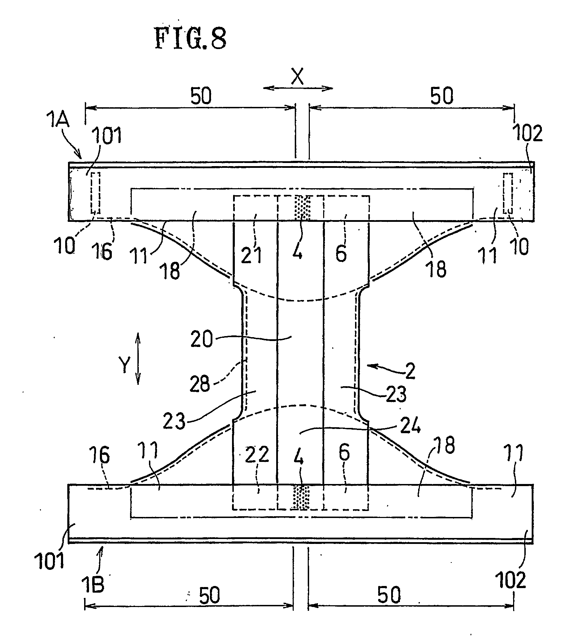

FIG. **1** is a schematic perspective view illustrating a diaper according to a first embodiment of the present invention, FIG. **2** is a partially cutaway perspective view illustrating an important part of the diaper, FIG. **3** is a perspective view illustrating the diaper as it is worn, FIG. **4** is an exploded perspective view illustrating the components of the diaper being separated from one another, FIG. **5(a)** is a plan view illustrating the diaper being stretched in three directions, FIG. **5(b)** is a bottom view illustrating the same, FIG. **6(a)** and FIG. **6(b)** are cross-sectional views taken along lines VIa-VIa and VIb-VIb, respectively, in FIG. **1**, FIG. **7(a)** is a cross-sectional view taken along line VIIa-VIIa in FIG. **1**, FIG. **7(b)** and FIG. **7(c)** are cross-sectional views taken along the same line illustrating alternative embodiments, and FIG. **8** is a schematic plan view illustrating a diaper according to a second embodiment being spread out.

### BEST MODE FOR CARRYING OUT THE INVENTION

The embodiments of the present invention will now be described with reference to the drawings.

FIG. **1** to FIG. **7(c)** illustrate a disposable diaper according to the first embodiment.

As illustrated in FIG. **1** and FIG. **3**, the diaper of the present embodiment includes a waist portion **1** that fits around the waist of the wearer, and a core portion **2** covering the crotch of the wearer. The waist portion **1** and the core portion **2** may be attached together in a T-shaped pattern in which the portions are arranged generally perpendicular to each other.

The core portion **2** of FIG. **1** includes a central portion **20,** and first and second end portions **21** and **22** located at opposite ends of the core portion **2** in the longitudinal direction Y. The first end portion **21** is attached to the waist portion **1.** An attachment sheet **3** illustrated in FIG. **5(b)** may be bonded on the outer surface of the second end portion **22.** Moreover, a holding sheet **3a** for holding down end portions of a pair of cuffs **23** may be bonded on the inner surface of the second end portion **22** of FIG. **5(a)**. An attachment member **10** to be in a planar engagement with the attachment sheet **3** is attached on the inner surface of each end portion of the waist portion **1**. The attachment member **10** may include, for example, the hooks of a hook-and-loop fastener, in which case the waist portion **1** can be attached to, and detached from, the second end portion **22** of the core portion **2** via the attachment sheet **3** of FIG. **5(b)**.

As illustrated in FIG. **4**, the core portion **2** includes the pair of cuffs **23** to be in contact with the surface of the wearer, a liquid-permeable topsheet **24,** a liquid-absorbing absorbent **25,** and a liquid-impermeable backsheet **26.** Moreover, the core portion **2** may include a first outer material **27** and/or a first leg elastic member **28**. The cuff **23** may include a cuff elastic member **29** for contracting the cuff **23** in the Y direction. Note that the backsheet **26** may be a sheet that is air-permeable and water-repellent.

FIG. **6(a),** FIG. **6(b)** and FIG. **7(a)** are schematic cross-sectional views taken along lines VIa-VIa, VIb-VIb and VIIa-VIIa, respectively, in FIG. **1.**

In these schematic views, each dotted area represents an area on which an adhesive is applied. The adhesive may be a hot-melt adhesive. Note that where a hot-melt adhesive is used as the adhesive, the application of the adhesive may be performed by using a bead coater, a coater, a spiral coater, a curtain coater, a spray coater, a transfer roll, or the like. The hot-melt adhesive may be a synthetic rubber resin, an olefin resin, or the like. Moreover, an adhesive may be applied on an elastic member.

As illustrated in FIG. **6(a)**, the backsheet **26** and the first outer material **27** are layered and bonded together with the first leg elastic member **28** being interposed therebetween. The backsheet **26** and the topsheet **24** are layered and bonded together into a single piece with the absorbent **25** being interposed therebetween. Note that a portion of the cuff **23** is bonded to the inner surface of the backsheet **26**. The leg elastic member **28** may be interposed between the topsheet **24** and the backsheet **26**. Alternatively, the leg elastic member **28** may be interposed between the cuff **23** and the topsheet **24**, the backsheet **26** or the first outer material **27**.

The core portion **2**, which includes layers stacked on one another as described above, is bonded to the waist portion **1** of FIG. **1** as will be described later.

As illustrated in FIG. **4**, the waist portion **1** includes a cover material **11** (an example of the third sheet) to be in contact with the surface of the wearer, an inner material **12** (an example of the second sheet), an outer material **14** (an example of the first sheet) and a plurality of waist elastic members **13.** The waist elastic members **13** are interposed between the inner material **12** and the outer material **14**. A cover elastic member **15** for contracting the cover material **11** in the longitudinal direction X of the waist portion **1** may be bonded to the cover material **11**. The outer material **14** may include a second leg elastic member **16.** The waist elastic members **13** contract the waist portion **1** in the longitudinal direction X, thereby forming waist gathers **Wg** (FIG. **1**) in the waist portion **1** of the diaper. The inner material **12** and the outer material **14** may be integrally provided with protruding portions **12a** and **14a**, respectively, which protrude toward the central portion **20** of the core portion **2**.

Note that the waist elastic member **13**, the cover elastic member **15**, the second leg elastic member **16**, the first leg elastic member **28** and the cuff elastic member **29** may each be a rubber thread. Where the waist elastic members **13** are rubber threads, the rubber threads may be arranged parallel to one another.

As illustrated in FIG. **6(a)**, the inner material **12** and the outer material **14** are layered and bonded together with the waist elastic members **13** being interposed therebetween. As illustrated in FIG. **6(a)** and FIG. **6(b),** a portion of the cover material **11** may be layered and bonded on the upper surface of the inner material **12**, forming a bag-like portion **18** in the area where the inner material **12** and the cover material **11** are not bonded together. The first end portion **21** of the core portion **2** illustrated in FIG. **1** and FIG. **2** is inserted into the bag-like portion **18**, and the first end portion **21** is interposed between the cover material **11** and the inner material **12**.

Now, the attachment between the waist portion **1** and the core portion **2** will be described.

The waist portion **1** and the first end portion **21** of the core portion **2** may be bonded together at a main bonding section **4** near the center of thewaist portion **1** in the longitudinal direction X, as indicated by dots in FIG. **6(a).** As indicated by dots in FIG. **4** and FIG. **1,** the main bonding section **4** is positioned near the center of the core portion **2** in the width direction X. Therefore, as illustrated in the partially cutaway perspective view of FIG. **2**, the waist portion **1** and the first end portion **21** of the core portion **2** may not be bonded together at non-bonding sections **6** on the left and right of the main bonding section **4** (FIG. **1**). Moreover, the bag-like portion **18** has an opening facing toward the central portion **20** of the core portion **2.** Note that the bag-like portion **18** may be bonded to the core portion **2** at a bonding section **4a** located around a position above the main bonding section **4.**

Moreover, as indicated by dots in FIG. **1** and FIG. **4,** the inner material **12** and the first outer material **27** may be bonded together at an auxiliary bonding section **7** near the central portion **20** of the core portion **2**. At such a position, there is no influence of the contraction force from the waist elastic members **13** forming the waist gathers **Wg**. Therefore, problems such as a stiff feel due to contraction will not occur in the core portion **21.**

FIG. **5(a)** and FIG. **5(b)** are a plan view and a bottom view, respectively, illustrating the diaper of the present embodiment being stretched in three directions (in a spread-out position) against the contraction force from the elastic members.

In the spread-out position of FIG. **5(a)**, the bag-like portion **18** has a width in the X direction that is substantially larger than that of the core portion **2**, as indicated by a two-dot chain line, whereby the width of the bag-like portion **18** remains to be larger than that of the core portion **2** even when the waist portion **1** of FIG. **1** is in a contracted position. Therefore, the first end portion **21** of the core portion **2** can be housed in the bag-like portion **18** without being contracted in the width direction X.

The waist elastic members **13** may be divided at a position in the longitudinal direction X so that they do not extend entirely across the main bonding section **4** of FIG. **4.** Thus, in the present embodiment, the waist elastic members **13** are not provided generally across the entirety of the main bonding section **4** (but are provided only in portions of the main bonding section **4**), and are provided only in areas **50** illustrated in FIG. **5(a).** Therefore, as illustrated in FIG. **1** and FIG. **3,** the waist elastic members **13** contract the waist portion **1** in the X direction, except in the main bonding section **4** and end portions of the waist portion **1**. Note that as illustrated in FIG. **4,** the waist elastic members **13** are also provided above the bag-like portion **18** so as to extend parallel to the bag-like portion **18,** thus contracting the waist portion **1** in the X direction, except in the main bonding section **4.**

Moreover, the cover elastic member **15** of FIG. **4** may be continuous generally across the entire length of the waist portion **1** in the longitudinal direction of the waist portion **1** along the edge of the opening of the bag-like portion **18** (FIG. **1**). Thus, the cover elastic member **15** contracts the cover material **11**.

In the diaper of the present embodiment, the waist gathers **Wg** are formed as illustrated in FIG. **1** by the contraction force from the waist elastic members **13** of FIG. **4**. However, the core portion **2** is unlikely to be contracted (distorted) in the width direction X because the contraction force from the waist elastic members **13** is reduced in the main bonding section **4.** Therefore, the core portion **2** is kept in a near-flat position, whereby the core portion **2** will not give a stiff feel, thus providing an improved wearability.

Moreover, since the core portion **2** can transform according to the movement of the wearer, thus providing an improved fit.

The waist gathers **Wg** can contract/expand freely without being restricted by the core portion **2,** thereby increasing the facility in putting the diaper on the wearer while also providing an improved fit to the waist.

As clearly shown in FIG. **2**, the first end portion **21** of the core portion **2** includes the non-bonding sections **6,** which may be housed in the bag-like portion **18** of FIG. **1**. Thus, the core portion **2** is bonded to the waist portion **1** only in the bonding section **4** at the center in the left-right direction. However, since the non-bonding section **6** is covered by the cover material **11**, the non-bonding section **6** will not be turned up, be kinked or slip out of position.

The cover material **11** is slightly apart from the core portion **2** in the bag-like portion **18** and has the cover elastic member **15** (FIG. **4**), whereby the cover material **11** will be in contact with the skin below the waist, thus serving as a anti-leak cuff. Thus, the cover material **11** suppresses a leak of bodily discharge through the waist portion.

Where the protruding portions **12a** and **14a** are provided as illustrated in FIG. **4,** the second leg elastic member **16** and the first leg elastic member **28** may be line up with each other as illustrated in FIG. **5(a)** and FIG. **5(b).** At least one second leg elastic member **16** may be arranged along the edge of the outer material **14**. For example, the second leg elastic member **16** may be arranged entirely across a portion of the outer material **14** for holding the waist and the opposing portion. Moreover, of the second leg elastic member **16** arranged across the entire article, a portion that overlaps with the core portion **2** may be cut off. By cutting off the elastic member as described above, the core portion **2** will not be contracted. Note that even when the second leg elastic member **16** is arranged in a portion of the outer material **14** that corresponds to the core portion **2**, the presence of the second leg elastic member **16** will not cause a problem if the contraction force from the second leg elastic member **16** is weaker than those from other portions. For example, the tension of the second leg elastic member **16** may be controlled during the process of interposing the second leg elastic member **16** between the outer material **14** and the inner material **12**, so as to weaken the contraction force from the portion of the second leg elastic member **16** that corresponds to the core portion **2**.

Note that instead of controlling the tension thereof, the portion of the second leg elastic member **16** that corresponds to the core portion **2** may be embossed so as to reduce the contraction force from the second leg elastic member **16**.

At least one first leg elastic member **28** may be arranged in the longitudinal direction Y of the core portion **2** and along each side edge of the first outer material **27**. The first leg elastic member **28** arranged entirely along the side edge of the first outer material **14** may be cut off in a portion thereof that corresponds to the waist portion **1**. By cutting off the elastic member as described above, the waist portion **1** will not be contracted. Note that even when the first leg elastic member **28** is arranged in a portion of the first outer material **27** that corresponds to the waist portion **1,** the presence of the first leg elastic member **28** will not cause a problem if the contraction force from the first leg elastic member **28** is weaker than those from other portions. For example, the tension of the first leg elastic member **28** may be controlled during the process of interposing the first leg elastic member **28** between the first outer material **27** and the backsheet **26**, so as to weaken the contraction force from the portion of the first leg elastic member **28** that corresponds to the waist portion **1**.

Note that instead of controlling the tension thereof, the portion of the first leg elastic member **28** that corresponds to the waist portion **1** may be embossed so as to reduce the contraction force from the first leg elastic member **28**.

Moreover, pre-cut pieces of the first leg elastic member **28** may be attached to the core portion **2** while being stretched so as to arrange the pieces of the first leg elastic member **28** at predetermined positions on the core portion **2**. The second leg elastic member **16** and the first leg elastic member **28** form leg gathers **Lg** in the diaper, as illustrated in FIG. **1**, FIG. **2** and FIG. **3**.

Note that an upper edge portion of the outer material **14** may be folded back onto, and attached to, the cover material **11**, as illustrated in FIG. **7(a)**. Alternatively, the outer material **14** may be layered together with the inner material **12** and the cover material **11**, instead of being folded back, as illustrated in FIG. **7(b)**. Alternatively, a single sheet may be folded back to form the cover material **11** and the outer material **14**.

As illustrated in FIG. **8,** the diaper of the present embodiment may include first and second waist portions **1A** and **1B** attached to the first and second end portions **21** and **22,** respectively, of the core portion **2.** The first and second waist portions **1A** and **1B** have a structure that is similar to or the same as that of the waist portion **1.** Note that elastic members for forming waist gathers are not shown in FIG. **8.** The core portion **2** may be folded in two, with the first and second waist portions **1A** and **1B** being placed onto each other, and with end portions **101** and **102** of the first waist portion **1A** being attached to those of the second waist portion **1B**. Through such an attachment, it is possible to form a pants-type diaper. The attachment of the end portions **101** and **102** for forming a pants-type diaper may be done through heat sealing or ultrasonic sealing.

Moreover, a plurality of hook-and-loop fasteners or adhesive tapes may be provided on the outer surface of the first waist portion **1A**. After disengagement of the end portions **101** and **102,** the hook-and-loop fasteners can be attached to the outer surface of the second waist portion **1B.**

While preferred embodiments of the present invention have been described above with reference to the drawings, obvious variations and modifications will readily occur to those skilled in the art upon reading the present specification.

For example, in the embodiment described above, the first end portion **21** of the core portion **2** of FIG. **1** may have a narrow width corresponding to that of the bonding section **4,** and the entirety of the first end portion **21** may be bonded to the waist portion **1**. Moreover, the entirety of the first end portion **21** of FIG. **1**, having a large width, may be bonded to the waist portion **1**.

Moreover, it is not necessary that the inner material **12** and the outer material **14** of the waist portion **1** include the protruding portions **12a** and **14a**.

The present invention may be applicable to disposable pants, as well as to disposable diapers.

Thus, such variations and modifications shall fall within the scope of the present invention as defined by the appended claims.

### INDUSTRIAL APPLICABILITY

The present invention can be employed in the structure of disposable diapers or disposable pants.

## Claims

1. A disposable worn article, comprising: a core portion including a first end portion, a central portion and a second end portion; and a waist portion crossing the core portion, wherein:
the waist portion includes an elastic member for contracting/expanding the waist portion in a longitudinal direction of the waist portion, and first and second sheets interposing the elastic member therebetween;
a portion of the waist portion and a portion of the first end portion of the core portion are bonded together in a bonding section; and
a contraction force from the elastic member is reduced or inactivated in the bonding section.

2. A disposable worn article, comprising: a core portion suitable for covering a crotch of a wearer; and a waist portion capable of fitting to a waist of the wearer, wherein:
the core portion includes a central portion with an absorbent, and first and second end portions located on opposite longitudinal ends of the core portion with respect to the central portion;
the waist portion is attached to the first end portion so as to cross the core portion;
the waist portion includes an elastic member for contracting/expanding the waist portion in a width direction of the core portion;
a portion of the waist portion and a portion of the first end portion of the core portion are bonded together in a bonding section; and
a contraction force in the bonding section is smaller than a contraction force from waist gathers formed by the elastic member.

3. A disposable worn article according to claim 1 or 2, wherein the elastic member is provided in a longitudinal direction of the waist portion and is divided at at least one position in the longitudinal direction so as not to extend entirely across the bonding section.

4. A disposable worn article according to claim 1, 2 or 3, wherein a portion of the elastic member is solidified after being melted in the bonding section.

5. A disposable worn article according to claim 1, 2, 3 or 4, wherein:
the waist portion includes first, second and third sheets layered on one another; and
the first end portion of the core portion is interposed between the second sheet and the third sheet.

6. A disposable worn article according to claim 5, wherein a portion of the first end portion of the core portion is bonded to a portion of at least one of the second sheet and the third sheet.

7. A disposable worn article according to any one of claims 1 to 6, wherein:
the disposable worn article further comprises leg gathers;
a portion of the leg gathers is provided in the core portion; and
another portion of the leg gathers is provided in the waist portion.

8. A disposable worn article according to any one of claims 1 to 7, further comprising an attachment member for allowing the waist portion and the second end portion of the core portion to be attached to each other.

9. A disposable worn article according to any one of claims 1 to 7, wherein:
the disposable worn article further comprises another waist portion crossing the core portion and including another elastic member;
first ends of the pair of waist portions are sealed together; and
second ends of the pair of waist portions are sealed together.
